# EUROPEAN PATENT SPECIFICATION

(11) **EP 4 578 020 B1**
(45) Date of publication and mention of the grant of the patent: **14.10.2026**
(21) Application number: 23772657.5
(22) Date of filing: 24.08.2023
(51) Int. Cl.: G16H 20/10, G16H 20/40, G01N 33/68, G16H 50/20, G16H 50/30

(54) **USE OF CARDIAC TROPONIN AND GALECTIN-3 TO DIFFERENTIATE MYOCARDIAL INFARCTION TYPE I AND TYPE II**
VERWENDUNG VON HERZTROPONIN UND GALECTIN-3 ZUR DIFFERENZIERUNG VON MYOKARDINFARKT TYP I UND TYP II
UTILISATION DE LA TROPONINE CARDIAQUE ET DE LA GALECTINE-3 POUR DIFFERENCIER L'INFARCTUS DU MYOCARDE DE TYPE I ET DE TYPE II

(30) Priority: 26.08.2022 US 202263401335 P; 05.05.2023 US 202363464412 P
(43) Date of publication of application: 02.07.2025
(73) Proprietor: Abbott Laboratories, Abbott Park, IL 60064 (US)
(72) Inventor: MURTAGH, Gillian, Abbott Park, Illinois 60064 (US); JACKSON, Laurel, Abbott Park, Illinois 60064 (US); MAHLER, Simon, Winston-salem, North Carolina 27157 (US); SNAVELY, Anna, Winston-salem, North Carolina 27157 (US); MILLER, Chadwick, Winston-salem, North Carolina 27157 (US)
(74) Representative: Carpmaels & Ransford LLP
(86) International application number: PCT/US2023/031006
(87) International publication number: WO 2024/044288

(56) References cited:
- US-A1- 2022 202 342
- DOUDESIS D ET AL: "Validation of the myocardial-ischaemic-injury-index machine learning algorithm to guide the diagnosis of myocardial infarction in a heterogenous population: a prespecified exploratory analysis", THE LANCET, DIGITAL HEALTH, 4 May 2022 (2022-05-04), pages e300 - e308, XP093086390
- NAGLAH A ET AL: "Ensemble Learning of Blood Metabolic Biomarkers: A Novel CAD System of Heart Failure", 2019 FIFTH INTERNATIONAL CONFERENCE ON ADVANCES IN BIOMEDICAL ENGINEERING (ICABME), IEEE, 17 October 2019 (2019-10-17), pages 1 - 4, XP033679071
- MILLARD R W ET AL: "Complementary, Alternative, and Putative Nontroponin Biomarkers of Acute Coronary Syndrome: New Resources for Future Risk Assessment Calculators", REVISTA ESPAÑOLA DE CARDIOLOGÍA (ENGLISH EDITION), vol. 67, no. 4, 11 March 2014 (2014-03-11), pages 312 - 320, XP028834344, ISSN: 1885-5857, DOI: 10.1016/J.REC.2013.12.011
- BACKUS B E ET AL: "A prospective validation of the HEART score for chest pain patients at the emergency department", INTERNATIONAL JOURNAL OF CARDIOLOGY, ELSEVIER, AMSTERDAM, NL, vol. 168, no. 3, 7 March 2013 (2013-03-07), pages 2153 - 2158, XP028736635, ISSN: 0167-5273, DOI: 10.1016/J.IJCARD.2013.01.255

## Description

### RELATED APPLICATION INFORMATION

This application claims priority to U.S. Application No. 63/401,335 filed on August 26, 2022 and U.S. Application No. 63/464,412, filed on May 5, 2023.

### FIELD OF THE INVENTION

The methods for determining whether a subject suspected of having a myocardial infarction is experiencing a Type I or Type II myocardial infarction. In particular, systems and methods are provided that employ a probability score based on decision tree based algorithms to process a subject's sex, age, and cardiac troponin concentration(s) along with subject's galectin-3 (Gal-3) concentration.

### BACKGROUND

Each year in the United States, over six million patients present to hospitals for evaluation of suspected Acute Coronary Syndrome (ACS). The most serious diagnosis associated with ACS, which usually presents with chest pain and associated symptoms, is myocardial infarction (MI). There are several types of MI. Type I is the classical type associated with rupture or erosion of a plaque. Type I MI usually causes platelet activation, thrombus formation and ultimately blockage of a coronary artery, stopping blood flow to the muscle (myocardium) supplied by that artery. Typically, patients diagnosed with Type I MI are brought to the catheterization lab immediately for a coronary angiogram with or without percutaneous coronary intervention (PCI; balloon and stenting) or less frequently to surgery for coronary artery bypass grafting (CABG) if necessary.

Type II MI is most often due to oxygen supply/demand imbalance in the myocardium, with or without atherosclerosis and vascular endothelial dysfunction, where the demand of the myocardium for oxygen increases but cannot be met by the supply. The increase in demand is caused by issues like sepsis, severe anemia, and/or abnormal heart rhythms. Treatment of Type II MI generally involves addressing the underlying pathological cause. PCI or CABG alone is less likely to be effective since the problem is not being primarily caused by a blocked artery.

Due to the differences in etiologies and treatments, differentiating between MI types is critical to offer the best care to the patient. Currently the distinction is usually based on clinical and electrocardiogram (EKG) criteria which are not always accurate.

US2022/202342Al discloses methods for reporting an estimated risk of ACS and/or an ACS comorbidity in a subject, wherein the ACS is selected from a group comprising Type I myocardial infarction or Type II myocardial infarction.

### SUMMARY OF THE INVENTION

Provided herein are methods for determining whether a subject suspected of having a myocardial infarction is experiencing a Type I myocardial infarction or a Type II myocardial infarction.

In some embodiments the methods comprise a) obtaining subject values for the subject, wherein said obtaining subject values comprises electronically receiving said subject values, wherein said subject values comprise: i) a subject sex value; ii) a subject age value; iii) a subject initial cardiac troponin concentration from an initial sample from the subject; and iv) a subject galectin-3 (Gal-3) concentration from an initial sample from the subject; b) processing said subject's sex, age, and cardiac troponin value with a processing system such that an algorithm index score is determined for said subject, wherein said processing system comprises: i) a computer processor, and ii) non-transitory computer memory comprising one or more computer programs and a database, wherein said one or more computer programs comprise an additive tree algorithm, wherein said database comprises at least M number of decision trees, wherein each individual decision tree comprises at least two pre-determined splitting variables and at least three pre-determined terminal node values, wherein said at least two pre-determined splitting variables are a threshold initial cardiac troponin concentration value, a sex value, and/or an age value, wherein said one or more computer programs, in conjunction with said computer processor, is/are configured to: i) apply said subject initial cardiac troponin concentration, said subject sex value, and/or said age value to said database to determine a terminal node value for each of said at least M number of decision trees, and ii) apply said additive tree algorithm to: (a) determine a combined value from M number of said terminal node values, and (b) process said combined value to determine the algorithm index score that the subject is experiencing a myocardial infarction, wherein M is an integer of at least 2; c) reporting said algorithm index score for the subject determined by said processing system; d) generating a probability score based on: i) the subject's Gal-3 concentration and ii) said algorithm index score; and e) determining whether the subject has a Type I myocardial infarction or a Type II myocardial infarction based on the probability score.

In some embodiments, the subject values further comprise a first, second or a first and second subsequent cardiac troponin concentration from corresponding first and/or second subsequent samples from the subject. In some embodiments, the at least two pre-determined splitting variables are: a threshold cardiac troponin rate of change value, a threshold initial cardiac troponin concentration value or a combination thereof; and a sex value and/or an age value. In some embodiments, the one or more computer programs, in conjunction with said computer processor, is/are further configured to apply said rate of change algorithm to determine a subject cardiac troponin rate of change value from at least two of: said subject initial cardiac troponin concentration, said first subsequent cardiac troponin concentration, and said second subsequent cardiac troponin concentration.

In some embodiments M is an integer from 2 to 1000. In other embodiments, M is an integer from 2 to 100,000. The integer selected for M will be determined based on the optimal number of trees for boosting the algorithm, which can be determined using routine techniques known in the art.

In some embodiments, the methods comprise: a) obtaining subject values for the subject, wherein said obtaining subject values comprises electronically receiving said subject values, wherein said subject values comprise: i) a subject sex value; ii) a subject age value; iii) a subject initial cardiac troponin concentration from an initial sample from the subject; iv) a subject galectin-3 (Gal-3) concentration from an initial sample from the subject; and v) a first, second or a first and second subsequent cardiac troponin concentration from corresponding first and/or second subsequent samples from the subject; b) processing said subject's sex, age, and cardiac troponin values with a processing system such that an algorithm index score is determined for said subject, wherein said processing system comprises: i) a computer processor, and ii) non-transitory computer memory comprising one or more computer programs and a database, wherein said one or more computer programs comprise: a rate of change algorithm and an additive tree algorithm, and wherein said database comprises at least M number of decision trees, wherein each individual decision tree comprises at least two pre-determined splitting variables and at least three pre-determined terminal node values, wherein said at least two pre-determined splitting variables are: a threshold cardiac troponin rate of change value, a threshold initial cardiac troponin concentration value or a combination thereof; and a sex value and/or an age value, wherein said one or more computer programs, in conjunction with said computer processor, is/are configured to: i) apply said rate of change algorithm to determine a subject cardiac troponin rate of change value from at least two of: said subject initial cardiac troponin concentration, said first subsequent cardiac troponin concentration, and said second subsequent cardiac troponin concentration, ii) apply said subject cardiac troponin rate of change value, said subject initial cardiac troponin concentration, said subject sex value, and/or said age value to said database to determine a terminal node value for each of said at least M number of decision trees, and iii) apply said additive tree algorithm to: (a) determine a combined value from M number of said terminal node values, and (b) process said combined value to determine the algorithm index score that the subject is experiencing a myocardial infarction; wherein M is an integer of at least 2, and c) reporting said algorithm index score for the subject determined by said processing system; d) generating a probability score based on: i) the subject's Gal-3 concentration and ii) said algorithm index score; and e) determining whether the subject has a Type I myocardial infarction or a Type II myocardial infarction based on the probability score.

In some embodiments, the subject is determined to have a Type I myocardial infarction based on the probability score. In some embodiments, the subject is determined to have a Type II myocardial infarction based on the probability score.

In some embodiments, obtaining subject values comprises receiving said subject values from a testing lab, from an analytical testing system, and/or from a hand-held or point of care testing device. In some embodiments, said processing system further comprises said analytical testing system and/or said hand-held or point of care testing device.

Obtaining subject values comprises electronically receiving said subject values.

In some embodiments, the initial cardiac troponin concentration, the first cardiac troponin concentration and/or the second cardiac troponin concentration is obtained by performing a cardiac troponin detection assay. In some embodiments, said cardiac troponin detection assay comprises an immunoassay. In some embodiments, the cardiac troponin detection assay is a single molecule detection assay.

In some embodiments, the Gal-3 concentration is obtained by performing a Gal-3 detection assay. In some embodiments, said Gal-3 detection assay comprises an immunoassay. In some embodiments, the Gal-3 detection assay is a single molecule detection assay.

In some embodiments, the methods further comprise manually or automatically inputting said subject values into said processing system. In some embodiments, said subject values are input into said processing system using a combination of manual and automatic input. For example, age and/or sex may be input manually and Gal-3 concentration and/or cardiac troponin concentration are input automatically.

In some embodiments, the cardiac troponin is cardiac troponin I (cTnI). In some embodiments, the cardiac troponin is cardiac troponin T (cTnT). In some embodiments, the cardiac troponin is cTnI and cTnT.

In some embodiments, said subject is a human.

In some embodiments, said initial samples from said subject comprises a blood, serum, or plasma sample. In some embodiments, said first and/or second subsequent samples comprise blood, serum, or plasma samples.

In some embodiments, said M decision trees is at least 100 different decision trees. In some embodiments, said M decision trees is at least 800 different decision trees.

Other embodiments and embodiments of the disclosure will be apparent in light of the following detailed description and related figures.

In some embodiments, said initial samples from said subject comprises a blood, serum, or plasma sample. In some embodiments, said first and/or second subsequent samples comprise blood, serum, or plasma samples.

In some embodiments, said M decision trees is at least 100 different decision trees. In some embodiments, said M decision trees is at least 800 different decision trees.

Other embodiments and embodiments of the disclosure will be apparent in light of the following detailed description and related figures.

### BRIEF DESCRIPTION OF THE FIGURES

FIG. 1 is a graph of the area under the curve (AUC) comparison of the algorithm index score alone versus the algorithm index score plus baseline Gal-3 for Type I vs. Type II MI in population of n = 123 patients with available baseline troponin samples.
FIG. 2 is a graph of AUC comparison of the algorithm index score alone versus the algorithm index score plus baseline Gal-3 for Type I vs. Type II MI in population of n = 86 patients with available serial troponin samples.
FIG. 3 shows distribution plots of predicted probabilities from logistic regression for Gal-3 plus the baseline MI3 score with a horizontal line representing the optimal cutoff.
FIG. 4 shows distribution plots of predicted probabilities from logistic regression for Gal-3 plus serial MI3 score with a horizontal line representing the optimal cutoff.

### DETAILED DESCRIPTION

Previously an algorithm which takes a patient's age, sex, and two serial high-sensitivity troponin measurements was used to distinguish MI patients (either Type I alone, or Type I and II combined) from non-MI patients. Disclosed herein are methods applying the algorithm, both with an initial troponin measurements and serial troponin measurements, in addition to galectin-3 (Gal-3) concentrations for distinguishing Type I from Type II MI, thereby allowing for better patient management in identifying patients who are at the highest risk for complications and should receive invasive management.

### Definitions

The terms "comprise(s)," "include(s)," "having," "has," "can," "contain(s)," and variants thereof, as used herein, are intended to be open-ended transitional phrases, terms, or words that do not preclude the possibility of additional acts or structures. The singular forms "a," "and," and "the" include plural references unless the context clearly dictates otherwise. The present disclosure also contemplates other embodiments "comprising," "consisting of," and "consisting essentially of," the embodiments or elements presented herein, whether explicitly set forth or not.

For the recitation of numeric ranges herein, each intervening number there between with the same degree of precision is explicitly contemplated. For example, for the range of 6-9, the numbers 7 and 8 are contemplated in addition to 6 and 9, and for the range 6.0-7.0, the number 6.0, 6.1, 6.2, 6.3, 6.4, 6.5, 6.6, 6.7, 6.8, 6.9, and 7.0 are explicitly contemplated.

Unless otherwise defined herein, scientific, and technical terms used in connection with the present disclosure shall have the meanings that are commonly understood by those of ordinary skill in the art. The meaning and scope of the terms should be clear; in the event, however of any latent ambiguity, definitions provided herein take precedent over any dictionary or extrinsic definition. Further, unless otherwise required by context, singular terms shall include pluralities and plural terms shall include the singular.

The term "acute coronary syndrome" or "ACS" as used herein refers to a group of conditions due to decreased blood flow in the coronary arteries such that part of the heart muscle is unable to function properly or dies. The most common symptom is chest pain, often radiating to the left arm or angle of the jaw, pressure-like in character, and associated with nausea and sweating. ACS usually occurs as a result of one of three problems: S and T wave (ST) elevation myocardial infarction (STEMI), non-ST elevation myocardial infarction (NSTEMI), or unstable angina (Torres and Moayedi, 2007 Clin. Geriatr. Med. 23 (2): 307-25, v). These types are named according to the appearance of the electrocardiogram (EKG) as non-ST segment elevation myocardial infarction and ST segment elevation myocardial infarction. There can be some variation as to which forms of myocardial infarction (MI) are classified under acute coronary syndrome. ACS should be distinguished from stable angina, which develops during exertion and resolves at rest. In contrast with stable angina, unstable angina occurs suddenly, often at rest or with minimal exertion, or at lesser degrees of exertion than the individual's previous angina ("crescendo angina"). New onset angina is also considered unstable angina, since it suggests a new problem in a coronary artery. Though ACS is usually associated with coronary thrombosis, it can also be associated with cocaine use. Cardiac chest pain can also be precipitated by anemia, bradycardias (excessively slow heart rate) or tachycardias (excessively fast heart rate). The cardinal symptom of decreased blood flow to the heart is chest pain, experienced as tightness around the chest and radiating to the left arm and the left angle of the jaw. This may be associated with diaphoresis (sweating), nausea and vomiting, as well as shortness of breath. In many cases, the sensation is "atypical," with pain experienced in different ways or even being completely absent (which is more likely in female patients and those with diabetes). Some may report palpitations, anxiety or a sense of impending doom (angor animi) and a feeling of being acutely ill. Patients with chest-pain are entering the emergency rooms of hospitals very frequently. Chest-pain, however, can result from many causes: gastric discomfort (e.g., indigestion), pulmonary distress, pulmonary embolism, dyspnea, musculoskeletal pain (pulled muscles, bruises) indigestion, pneumothorax, cardiac non-coronary conditions, and acute coronary syndrome (ACS). As mentioned above, ACS is usually one of three diseases involving the coronary arteries: ST elevation myocardial infarction, non ST elevation myocardial infarction, or unstable angina. These types are named according to the appearance of the electrocardiogram (EKG) as non-ST segment elevation myocardial infarction (NSTEMI) and ST segment elevation myocardial infarction (STEMI). ACS is usually associated with coronary thrombosis. The physician has to decide if the patient is having a life threatening ACS or not. In the case of such a cardiac event, rapid treatment by opening up the occluded coronary artery is essential to prevent further loss of myocardial tissue.

As used herein, "suspected of having acute coronary syndrome" means a subject has at least one of the symptoms of acute coronary syndrome described above (e.g., chest pain, experienced as tightness around the chest often radiating to the left arm and the left angle of the jaw, diaphoresis (sweating), nausea and vomiting, shortness of breath).

A "subject" or "patient" may be human or non-human and may include, for example, animal strains or species used as "model systems" for research purposes, such a mouse model as described herein. Likewise, subject may include either adults or juveniles (e.g., children). Moreover, patient may mean any living organism, preferably a mammal (e.g., humans and non-humans) that may benefit from the administration of compositions contemplated herein. Examples of mammals include, but are not limited to, any member of the Mammalian class: humans, non-human primates such as chimpanzees, and other apes and monkey species; farm animals such as cattle, horses, sheep, goats, swine; domestic animals such as rabbits, dogs, and cats; laboratory animals including rodents, such as rats, mice and guinea pigs, and the like. Examples of non-mammals include, but are not limited to, birds, fish, and the like. In one embodiment, the mammal is a human.

Preferred methods and materials are described below, although methods and materials similar or equivalent to those described herein can be used in practice or testing of the present disclosure. The materials, methods, and examples disclosed herein are illustrative only and not intended to be limiting.

### Differentiating Myocardial Infarction Type I and Type II

The invention provides systems and methods for determining whether a subject suspected of having a myocardial infarction is experiencing a Type I myocardial infarction or a Type II myocardial infarction.

The disclosed methods employ two predictors for classifying type of myocardial infarction (MI): 1) an algorithm index score and 2) a galectin-3 concentration.

### 1. Algorithm Index Score

The first predictor is an algorithm index score. Any machine learning algorithm known in the art can be used in the methods of the present disclosure to generate the algorithm index score. In some embodiments, the machine learning algorithm is an adaptive index modeling (AIM) algorithm. In other embodiments, the machine learning algorithm is a random forest algorithm. In yet other embodiments, the at least one machine learning algorithm is a logistic regression algorithm. In select embodiment, the machine learning algorithm is an additive decision tree based algorithm.

The algorithm index score may be generated using methods as described in Than, M.P., et al., Circulation. 2019;140:899-909, U.S. Patent No. 11,147,498, and U.S. Patent Application No. 17/398,589.

In some embodiments, generation of the algorithm index score utilizes additive decision tree based algorithms to process a subject's cardiac troponin concentration, and optionally, a subject's first, second or a first and second subsequent cardiac troponin concentration, the subject's age, and the subject's sex to calculate the probability that a patient is experiencing a myocardial infarction (MI). These variable inputs are evaluated via a decision tree based statistical calculation to provide an estimation of how likely it is that a patient is experiencing Type I MI or Type II MI such that that a subject can be stratified into appropriate categories.

In particular embodiments, the systems and methods herein address the variable of timing between sample collection by determining the rate of change of cardiac troponin based on the exact time or nearly exact time (e.g., in minutes) of the first collection and the second collection of the sample from the subject.

The systems and methods herein, in certain embodiments, address the age variable by determining the impact of the age decile the patient falls into. In some embodiments, the subject age value is either the subject's age in years or a set value based on range of ages. In select embodiments, the set value is determined based on the following ranges: 0-29 years old, 30-39 years old, 40-49 years old, 50-59 years old, 60-69 years old, 70-79 years old, and 80 years or older.

The systems and methods herein, in some embodiments, addresses the sex difference by categorizing the patients into male and female sex profiles. In select embodiments, the sex value is one number for males (e.g., 1.0) and another number for females (e.g., 0).

In some embodiments, the systems and methods comprise a computer processor and a non-transitory computer memory component comprising: one or more computer programs configured to access a database, wherein the one or more computer programs comprise an additive tree algorithm and, optionally a rate of change algorithm, and wherein the database comprises at least M number of decision trees, wherein each individual decision tree comprises at least two (e.g., two, three, four, or more) pre-determined splitting variables and at least three (e.g., three, four, five, six, or more) pre-determined terminal node values, wherein the at least two pre-determined splitting variables are: a threshold initial cardiac troponin concentration value, a sex value, and/or an age value; or a threshold cardiac troponin rate of change value, a threshold initial cardiac troponin concentration value or a combination thereof, and a sex value and/or an age value, in conjunction with the computer processor, is/are configured to: i) apply said subject initial cardiac troponin concentration, said subject sex value, and/or said age value to said database to determine a terminal node value for each of said at least M number of decision trees or apply the rate of change algorithm to determine a subject and ii) apply said additive tree algorithm to: (a) determine a combined value from M number of said terminal node values, and (b) process said combined value to determine the algorithm index score that the subject is experiencing a myocardial infarction; or i) apply said rate of change algorithm to determine a subject cardiac troponin rate of change value from at least two of: said subject initial cardiac troponin concentration, said first subsequent cardiac troponin concentration, and said second subsequent cardiac troponin concentration, ii) apply said subject cardiac troponin rate of change value, said subject initial cardiac troponin concentration, said subject sex value, and/or said age value to said database to determine a terminal node value for each of said at least M number of decision trees, and iii) apply said additive tree algorithm to: (a) determine a combined value from M number of said terminal node values, and (b) process said combined value to determine the algorithm index score that the subject is experiencing a myocardial infarction. In certain embodiments, the non-transitory computer memory component further comprises the database.

The additive tree algorithm may comprise at least M number of decision trees. Each individual decision tree comprises at least two pre-determined splitting variables and at least three pre-determined terminal node values. M may be an integer of at least 2. In some embodiments M is an integer from 2 to 100,000. The integer selected for M will be determined based on the optimal number of trees for boosting the algorithm and can be determined using routine techniques known in the art. For example M can be from 10-100,000, 100-100,000, 200-100,000, 300-100,000, 400-100,000, 500-100,000, 600-100,000, 700-100,000, 800-100,000, 900-100,000, 1000-100,000, 2000-100,000, 3000-100,000, 4000-100,000, 5000-100,000, 6000-100,000, 7000-100,000, 8000-100,000, 9000-100,000, 10-90,000, 100-90,000, 200-90,000, 300-90,000, 400-90,000, 500-90,000, 600-90,000, 700-90,000, 800-90,000, 900-90,000, 1000-90,000, 2000-90,000, 3000-90,000, 4000-90,000, 5000-90,000, 6000-90,000, 7000-90,000, 8000-90,000, 9000-90,000, 10-80,000, 100-80,000, 200-80,000, 300-80,000, 400-80,000, 500-80,000, 600-80,000, 700-80,000, 800-80,000, 900-80,000, 1000-80,000, 2000-80,000, 3000-80,000, 4000-80,000, 5000-80,000, 6000-80,000, 7000-80,000, 8000-80,000, 9000-80,000, 10-70,000, 100-70,000, 200-70,000, 300-70,000, 400-70,000, 500-70,000, 600-70,000, 700-70,000, 800-70,000, 900-70,000, 1000-70,000, 2000-70,000, 3000-70,000, 4000-70,000, 5000-70,000, 6000-70,000, 7000-70,000, 8000-70,000, 9000-70,000, 10-60,000, 100-60,000, 200-60,000, 300-60,000, 400-60,000, 500-60,000, 600-60,000, 700-60,000, 800-60,000, 900-60,000, 1000-60,000, 2000-60,000, 3000-60,000, 4000-60,000, 5000-60,000, 6000-60,000, 7000-60,000, 8000-60,000, 9000-60,000, 10-50,000, 100-50,000, 200-50,000, 300-50,000, 400-50,000, 500-50,000, 600-50,000, 700-50,000, 800-50,000, 900-50,000, 1000-50,000, 2000-50,000, 3000-50,000, 4000-50,000, 5000-50,000, 6000-50,000, 7000-50,000, 8000-50,000, 9000-50,000, 10-40,000, 100-40,000, 200-40,000, 300-40,000, 400-40,000, 500-40,000, 600-40,000, 700-40,000, 800-40,000, 900-40,000, 1000-40,000, 2000-40,000, 3000-40,000, 4000-40,000, 5000-40,000, 6000-40,000, 7000-40,000, 8000-40,000, 9000-40,000, 10-30,000, 100-30,000, 200-30,000, 300-30,000, 400-30,000, 500-30,000, 600-30,000, 700-30,000, 800-30,000, 900-30,000, 1000-30,000, 2000-30,000, 3000-30,000, 4000-30,000, 5000-30,000, 6000-30,000, 7000-30,000, 8000-30,000, 9000-30,000, 10-20,000, 100-20,000, 200-20,000, 300-20,000, 400-20,000, 500-20,000, 600-20,000, 700-20,000, 800-20,000, 900-20,000, 1000-20,000, 2000-20,000, 3000-20,000, 4000-20,000, 5000-20,000, 6000-20,000, 7000-20,000, 8000-20,000, 9000-20,000, 10-10000, 100-10,000, 200-10,000, 300-10,000, 400-10,000, 500-10,000, 600-10,000, 700-10,000, 800-10,000, 900-10,000, 1000-10,000, 2000-10,000, 3000-10,000, 4000-10,000, 5000-10,000, 6000-10,000, 7000-100,00, 8000-10,000, 9000-10,000, 10-1000, 100-1000, 200-1000, 300-1000, 400-1000, 500-1000, 500-2000, 600-1000, 700-1000, 800-1000, 900-1000, 10-900, 100-900, 200-900, 300-900, 400-900, 500-900, 600-900, 700-900, 800-900, 10-800, 100-800, 200-800, 300-800, 400-800, 500-800, 600-800, 700-800, 10-700, 100-700, 200-700, 300-700, 400-700, 500-700, 600-700, 10-600, 100-600, 200-600, 300-600, 400-600, 500-600, 10-500, 100-500, 200-500, 300-500, 400-500, 10-400, 100-400, 200-400, 300-400, 10-300, 100-300, 200-300, 10-200, 100-200, or 10-100. In some embodiments, M is at least 100, at least 200, at least 300, at least 400, at least 500, at least 600, at least 700, at least 800, at least 900, at least 1000, at least 1500, or at least 2000. In some embodiments, M is 1, as the algorithm includes a single decision tree.

In some embodiments, the algorithm index score is based on a non-weighted or weighted combination of each of the node values. In further embodiments, the combined value from M number of terminal nodes is a weighted combined valued represented by the formula: ${\sum }_{i = 1}^{M} {a}_{i} {T}_{i} \left(X, {B}_{i}\right)$, where Tᵢ represents the individual decision trees, X represents the subject values, *B*ᵢ presents the at least two splitting variables, *a*ᵢ represents a weight value, and ${Σ}_{i = 1}^{M}$ represents summing together all of the M decision trees. To solve for the estimated index score, the combined value from M number of terminal nodes is further processed using the following equation: log $log \frac{p 1}{1 - p 1} = {\sum }_{i = 1}^{M} {a}_{i} {T}_{i} \left(X, {B}_{i}\right)$, where pl represents the estimated risk of ACS. In some aspects, such as in the example below, p1 is solved for as the algorithm index score.

In some aspects, the algorithm may generate hundreds or thousands of individual tree scores which are combined into a summation score (SS) and an algorithm index score using the following generic formula where y̅ represents the mean value of the outcome. $SS = \left(\frac{1}{2}\right) ∗ {\sum }_{i = 0}^{n = M} {score}_{i} + \left(\frac{1}{2}\right) ∗ ln \frac{1 + \overline{y}}{1 - \overline{y}}$ $Algorithm Index Score = \frac{1}{\left(1+{exp}^{\left(-2SS\right)}\right)}$

For example, the algorithm may generate 987 individual tree scores which are combined into a SS using the below formula and an algorithm index score using the formula provided above. $SS = \left(\frac{1}{2}\right) ∗ {\sum }_{i = 0}^{n = 986} {score}_{i} + \left(\frac{1}{2}\right) ∗ ln \frac{1 - 0.731828742117491}{1 + 0.731828742117491}$

In some embodiments, the pre-determined splitting variables and/or the predetermined terminal node values are empirically derived from analysis of population data. In other embodiments, the analysis of population data comprises employing a machine learning algorithm as described above. For example, the analysis of population data may comprise using an additive decision tree based algorithm.

In some embodiments, the at least two pre-determined splitting variables comprise a threshold initial cardiac troponin concentration value, a sex value, and/or an age value. Alternatively, in some embodiments, the at least two pre-determined splitting variables comprise: a threshold cardiac troponin rate of change value or a threshold initial cardiac troponin concentration value; and a sex value; and/or an age value. In some embodiments, the at least two pre-determined splitting variables are selected from the group consisting of: a threshold cardiac troponin rate of change value, a threshold initial cardiac troponin concentration value, a sex value, and an age value. Thus, in some embodiments, the computer programs further apply said additive tree algorithm to: apply said rate of change algorithm to determine a subject cardiac troponin rate of change value from at least two of: said subject initial cardiac troponin concentration, said first subsequent cardiac troponin concentration, and said second subsequent cardiac troponin concentration.

In some embodiments, the algorithm index score is a baseline algorithm index score. The baseline algorithm index score utilizes a subject's sex, age, and initial cardiac troponin concentration.

In some embodiments, the algorithm index score is a serial algorithm index score. The serial algorithm index score utilizes a subject's sex, age, initial cardiac troponin concentration, and a first subsequent, second subsequent or a first and second subsequent cardiac troponin concentration corresponding to subsequently taken samples. The methods may use any number of subsequent samples in addition to the first subsequent, or first and second subsequent samples. For example, a third subsequent, a fourth subsequent, a fifth subsequent, a sixth subsequent, a seventh subsequent, etc. The subsequent samples may be taken at any interval from minutes, to hours, to days after the previous sample.

In some embodiments, the algorithm index score is reported as a result from 0 to 100. For example, the algorithm index score may be originally generated on a scale from 0 to 1 but is multiplied by 100 to increase interpretability.

In some embodiments, the methods further comprise reporting the algorithm index score for the subject. In some embodiments, the processing system generates algorithm index score results and/or reports based on the analysis.

### 2. Probability Score

A galectin-3 concentration, along with the algorithm index score, allows generation of a probability score. Any machine learning algorithm known in the art can be used in the methods of the present disclosure to generate the probability score. In some embodiments, the machine learning algorithm is an adaptive index modeling (AIM) algorithm. In other embodiments, the machine learning algorithm is a random forest algorithm. In other embodiments, the machine learning algorithm is a boosted tree algorithm, a Naive Bayes classification, a support vector machine, K-nearest neighbors (KNN), K means clusters, a neural network, or any combinations thereof.

In yet other embodiments, the at least one machine learning algorithm is a regression algorithm (e.g., logistic regression).

In select embodiments, the machine learning algorithm is a logistic regression model. Using available statistical software, such as R, SPSS, Systat, STATA, Eviews, AMOS, SAS, Python, and Mplus, the algorithm index score and galectin-3 baseline concentration can be entered into a logistic regression model. Any suitable logistic regression model may be used, and the methods described herein are not limited in this respect. Predicted probabilities from the model are generated using the statistical software to give the predicted probability of a Type I MI.

The probability score provides insight into how likely it is that a patient is experiencing Type I MI (e.g., the probability of type I MI is modeled). To determine whether a subject is experiencing a Type I myocardial infarction or a Type II myocardial infarction, the probability score may be compared to a cutoff score. The minimum distance method to determine an optimal cutoff for the probability score which ranges from 0 to 1, can be used. For example, Type I MI may be above the cutoff score, whereas probability scores below the cutoff score represent Type II MI.

In some embodiments, a clinician or other medical personnel can compare the probability score for the subject with a cutoff score. The cutoff score can be provided in a product insert or other publication, or on a website or on a mobile device (e.g., such as through an app).

In some embodiments, the cutoff score is 0.01, 0.02, 0.03, 0.04, 0.05, 0.06, 0.07, 0.08, 0.09, 0.10, 0.11, 0.12, 0.13, 0.14, 0.15, 0.16, 0.17, 0.18, 0.19, 0.20, 0.21, 0.22, 0.23, 0.24, 0.25, 0.26, 0.27, 0.28, 0.29, 0.30, 0.31, 0.32, 0.33, 0.34, 0.35, 0.36, 0.37, 0.38, 0.39, 0.40, 0.41, 0.42, 0.43, 0.44, 0.45, 0.46, 0.47, 0.48, 0.49, 0.50, 0.51, 0.52, 0.53, 0.54, 0.55, 0.56, 0.57, 0.58, 0.59, 0.60, 0.61, 0.62, 0.63, 0.64, 0.65, 0.66, 0.67, 0.68, 0.69, 0.70, 0.71, 0.72, 0.73, 0.74, 0.75, 0.76, 0.77, 0.78, 0.79, 0.80, 0.81, 0.82, 0.83, 0.84, 0.85, 0.86, 0.87, 0.88, 0.89, 0.90, 0.91, 0.92, 0.93, 0.94, 0.95, 0.96, 0.97, 0.98, or 0.99. In select embodiments, the cutoff score is 0.10. In select embodiments, the cutoff score is 0.11. In select embodiments, the cutoff score is 0.12. In select embodiments, the cutoff score is 0.13. In select embodiments, the cutoff score is 0.14. In select embodiments, the cutoff score is 0.15. In select embodiments, the cutoff score is 0.16. In select embodiments, the cutoff score is 0.17. In select embodiments, the cutoff score is 0.18. In select embodiments, the cutoff score is 0.19. In select embodiments, the cutoff score is 0.20. In select embodiments, the cutoff score is0. 21. In select embodiments, the cutoff score is 0.22. In select embodiments, the cutoff score is 0.23. In select embodiments, the cutoff score is 0.24. In select embodiments, the cutoff score is 0.25. In select embodiments, the cutoff score is 0.26. In select embodiments, the cutoff score is 0.27. In select embodiments, the cutoff score is 0.28. In select embodiments, the cutoff score is 0.29. In select embodiments, the cutoff score is 0.30. In select embodiments, the cutoff score is 0.31. In select embodiments, the cutoff score is 0.32. In select embodiments, the cutoff score is 0.33. In select embodiments, the cutoff score is 0.34. In select embodiments, the cutoff score is 0.35. In select embodiments, the cutoff score is 0.36. In select embodiments, the cutoff score is 0.37. In select embodiments, the cutoff score is 0.38. In select embodiments, the cutoff score is 0.39. In select embodiments, the cutoff score is 0.40. 0.In select embodiments, the cutoff score is 0.42. In select embodiments, the cutoff score is 0.43. In select embodiments, the cutoff score is 0.44. In select embodiments, the cutoff score is 0.45. In select embodiments, the cutoff score is 0.46. In select embodiments, the cutoff score is 0.47. In select embodiments, the cutoff score is 0.48. In select embodiments, the cutoff score is 0.49. In select embodiments, the cutoff score is 0.50. In select embodiments, the cutoff score is 0.51. In select embodiments, the cutoff score is 0.52. In select embodiments, the cutoff score is 0.54. In select embodiments, the cutoff score is 0.55. In select embodiments, the cutoff score is 0.56. In select embodiments, the cutoff score is 0.57. In select embodiments, the cutoff score is 0.58. In select embodiments, the cutoff score is 0.59. In select embodiments, the cutoff score is 0.60. In select embodiments, the cutoff score is 0.61. In select embodiments, the cutoff score is 0.62. In select embodiments, the cutoff score is 0.63. In select embodiments, the cutoff score is 0.64. In select embodiments, the cutoff score is 0.65. In select embodiments, the cutoff score is 0.66. In select embodiments, the cutoff score is 0.67. In select embodiments, the cutoff score is 0.68. In select embodiments, the cutoff score is 0.69. In select embodiments, the cutoff score is 0.70. In select embodiments, the cutoff score is 0.71. In select embodiments, the cutoff score is 0.72. In select embodiments, the cutoff score is 0.73. In select embodiments, the cutoff score is 0.74. In select embodiments, the cutoff score is 0.75. In select embodiments, the cutoff score is 0.76. In select embodiments, the cutoff score is 0.77. In select embodiments, the cutoff score is 0.78. In select embodiments, the cutoff score is 0.79. In select embodiments, the cutoff score is 0.80. In select embodiments, the cutoff score is 0.81. In select embodiments, the cutoff score is 0.82. In select embodiments, the cutoff score is 0.83. In select embodiments, the cutoff score is 0.84. In select embodiments, the cutoff score is 0.85. In select embodiments, the cutoff score is 0.86. In select embodiments, the cutoff score is 0.87. In select embodiments, the cutoff score is 0.88. In select embodiments, the cutoff score is 0.89. In select embodiments, the cutoff score is 0.90.

Exemplary logistic regression analysis for the generation of a probability score is provided in Example 1.

### 3. Subject Values

In some embodiments the methods comprise obtaining a subject sex value; a subject age value; a subject initial cardiac troponin concentration from an initial sample from the subject; and a subject galectin-3 (Gal-3) concentration from an initial sample from the subject; and, optionally, a first, second or a first and second subsequent cardiac troponin concentration from corresponding first and/or second subsequent samples from the subject.

The methods are not limited by the method of obtaining the subject values. In some embodiments, the methods comprise receiving said subject values from a testing lab, from

In select embodiments, the methods comprise receiving said subject values from an analytical testing system. In some embodiments, the processing system further comprises said analytical testing system. In some embodiments, the methods comprise receiving said subject values from a hand-held or point of care testing device. "Point-of-care device" refers to a device used to provide medical diagnostic testing at or near the point-of-care (namely, outside of a laboratory), at the time and place of patient care (such as in a hospital, physician's office, urgent or other medical care facility, a patient's home, a rehabilitation facility, nursing home or facility, an ambulance, a long-term care and/or hospice facility, or a subject's home or place of work). Such point-of-care devices can also include portable, desktop sized devices. Examples of point-of-care devices include those produced by Abbott Laboratories (Abbott Park, IL) (e.g., i-STAT^{®}, i-STAT^{®} Alinity, ID Now^{®}), Universal Biosensors (Rowville, Australia) (see US 2006/0134713), Axis-Shield PoC AS (Oslo, Norway) and Clinical Lab Products (Los Angeles, USA). As such, in some embodiments, the processing system further comprises a hand-held or point-of-care testing device.

The methods comprise obtaining subject values electronically. In some embodiments, the methods comprise manually inputting said subject values into said processing system. In some embodiments, the methods comprise automatically inputting said subject values into said processing system.

### 4. Biological Samples

Biological samples from a subject are tested to determine the concentration of cardiac troponin and galectin-3. Biological samples include, but are not necessarily limited to, bodily fluids such as blood-related samples (e.g., whole blood, serum, plasma, and other blood-derived samples), urine, cerebral spinal fluid, bronchoalveolar lavage, and the like. Another example of a biological sample is a tissue sample. A biological sample may be fresh or stored (e.g., blood or blood fraction stored in a blood bank). The biological sample may be a bodily fluid expressly obtained for the assays of this invention or a bodily fluid obtained for another purpose which can be sub-sampled for the assays of this invention. In certain embodiments, the biological sample is whole blood. Whole blood may be obtained from the subject using standard clinical procedures. In other embodiments, the biological sample is plasma. Plasma may be obtained from whole blood samples by known means, including but not limited to, centrifugation (e.g., of anti-coagulated blood), membrane- or filter-based separation, agglutination-based plasma separation, acoustic force, and microfluidics. Such process provides a buffy coat of white cell components and a supernatant of the plasma. In certain embodiments, the biological sample is serum. Serum may be obtained by centrifugation of whole blood samples that have been collected in tubes that are free of anti-coagulant. The blood is permitted to clot prior to centrifugation. The yellowish-reddish fluid that is obtained whole blood samples that have been collected in tubes that are free of anti-coagulant. The blood is permitted to clot prior to centrifugation. The yellowish-reddish fluid that is obtained by centrifugation is the serum. In another embodiment, the sample is urine. The sample may be pretreated as necessary by dilution in an appropriate buffer solution, heparinized, concentrated if desired, or fractionated by any number of methods including but not limited to ultracentrifugation, fractionation by fast performance liquid chromatography (FPLC), or precipitation of apolipoprotein B containing proteins with dextran sulfate or other methods. Any of a number of standard aqueous buffer solutions at physiological pH, such as phosphate, Tris, or the like, can be used.

In some embodiments, the initial samples are blood, serum, or plasma sample. In some embodiments, first and/or second subsequent samples comprise blood, serum, or plasma samples.

The sample can be obtained using techniques known to those skilled in the art, and the sample may be used directly as obtained from the source or following a pretreatment to modify the character of the sample. Such pretreatment may include, for example, preparing plasma from blood, diluting viscous fluids, filtration, precipitation, dilution, distillation, mixing, concentration, inactivation of interfering components, the addition of reagents, lysing, and the like.

The samples may be obtained in a medical facility, e.g., at an Emergency Room, urgent care clinic, walk-in clinic, a long term care facility, ambulatory clinic, rehabilitation facility, nursing facility, an ambulance, or another appropriate site of medical practice. The sample may be obtained in a home or residential setting (e.g., a senior living (e.g., facility) or hospice setting), or place of work, at the site of the suspected myocardial infarction, or during transportation to a medical facility (e.g., ambulance).

### 5. Detection Assays

The present invention is not limited by the type of assay used to detect and/or quantify cardiac troponin or galectin-3 (Gal-3).

In certain embodiments, an immunoassay is employed for detecting cardiac troponin and/or Gal-3. Any suitable assay known in the art can be used, including commercially available cardiac troponin or Gal-3 assays. Examples of such assays include, but are not limited to, immunoassay, such as sandwich immunoassay (e.g., monoclonal-polyclonal sandwich immunoassays, including radioisotope detection (radioimmunoassay (RIA)) and enzyme detection (enzyme immunoassay (ETA) or enzyme-linked immunosorbent assay (ELISA) (e.g., Quantikine ELISA assays, R&D Systems, Minneapolis, Minn.)), competitive inhibition immunoassay (e.g., forward and reverse), fluorescence polarization immunoassay (FPIA), enzyme multiplied immunoassay technique (EMIT), bioluminescence resonance energy transfer (BRET), and homogeneous chemiluminescent assay, one-step antibody detection assay, homogeneous assay, heterogeneous assay, capture on the fly assay, single molecule detection assay, lateral flow assay, etc.

Cardiac troponin and/or Gal-3 can be detected or quantified in a sample with the help of one or more separation methods. For example, suitable separation methods may include a mass spectrometry method, such as electrospray ionization mass spectrometry (ESI-MS), ESI-MS/MS, ESI-MS/(MS)ⁿ (n is an integer greater than zero), matrix-assisted laser desorption ionization time-of-flight mass spectrometry (MALDI-TOF-MS), surface-enhanced laser desorption/ionization time-of-flight mass spectrometry (SELDI-TOF-MS), desorption/ionization on silicon (DIOS), secondary ion mass spectrometry (SIMS), quadrupole time-of-flight (Q-TOF), atmospheric pressure chemical ionization mass spectrometry (APCI-MS), APCI-MS/MS, APCI-(MS)ⁿ, or atmospheric pressure photoionization mass spectrometry (APPI-MS), APPI-MS/MS, and APPI-(MS)ⁿ. Other suitable separation methods include chemical extraction partitioning, column chromatography, ion exchange chromatography, hydrophobic (reverse phase) liquid chromatography, isoelectric focusing, one-dimensional polyacrylamide gel electrophoresis (PAGE), two-dimensional polyacrylamide gel electrophoresis (2D-PAGE), or other chromatographic techniques, such as thin-layer, gas or liquid chromatography, or any combination thereof. In one embodiment, the biological sample to be assayed may be fractionated prior to application of the separation method.

The nature of methods and the test can be any assay known in the art such as, for example, immunoassays, point-of-care assays, clinical chemistry assay, protein immunoprecipitation, immunoelectrophoresis, chemical analysis, SDS-PAGE and Western blot analysis, or protein immunostaining, electrophoresis analysis, a protein assay, a competitive binding assay, a lateral flow assay, a functional protein assay, or chromatography or spectrometry methods, such as high-performance liquid chromatography (HPLC) or liquid chromatography-mass spectrometry (LC/MS). Also, the assay can be employed in a clinical chemistry format such as would be known by one of ordinary skill in the art.

Determining the concentration of cardiac troponin or galectin-3 by an immunoassay can be adapted for use in a variety of automated and semi-automated systems or platforms (including those wherein the solid phase comprises a microparticle) known in the art. The following adaptations of automated and/or semi-automated systems are included herein as merely exemplary. Specifically, the methods can utilize automated and semi-automated systems or platforms such as those described, e.g., U.S. Patent No. 5,063,081, U.S. Patent Application Publication Nos. 2003/0170881, 2004/0018577, 2005/0054078, and 2006/0160164 and as commercially marketed *e.g.,* by Abbott Laboratories (Abbott Park, IL) as Abbott Point of Care (i-STAT^{®} or i-STAT Alinity, ID Now^{®}, Abbott Laboratories) as well as those described in U.S. Patent Nos. 5,089,424 and 5,006,309, and as commercially marketed, e.g., by Abbott Laboratories (Abbott Park, IL) as ARCHITECT^{®} or the series of Abbott Alinity devices.

Other methods of detection include the use of or can be adapted for use on a nanopore device or nanowell device, e.g., for single molecule detection. As used herein the term "single molecule detection" refers to the detection and/or measurement of a single molecule of an analyte in a test sample at very low levels of concentration (such as pg/mL or femtogram/mL levels). A number of different single molecule analyzers or devices are known in the art and include nanopore and nanowell devices. Examples of nanopore devices are described in PCT International Application WO 2016/161402. Examples of nanowell device are described in PCT International Application WO 2016/161400.

In certain embodiments, the methods for detecting cardiac troponins T and I (cTnT and cTnI) are as described in U.S. Patent Application Publication 2012/0076803 and U.S. Patent Nos. 8,535,895, 8,8325,120. In certain embodiments, cTnI is detected with the ERENNA detection assay system from Singulex Inc. or Abbott's hs cTnI STAT ARCHITECT assay. In certain embodiments, the methods for detecting troponin T employ the Elecsys^{®} Troponin T high sensitive (TnT-hs) assay (ROCHE) (see, Li et al., Arch Cardiovasc Dis. 2016 March; 109(3):163-70)

Determining the level of galectin-3 in a subject typically includes measuring levels of polypeptide using methods known in the art and/or described herein, e.g., immunoassays, such as enzyme-linked immunosorbent assays (ELISA). One exemplary ELISA kit that is commercially available is the galectin-3 ELISA kit available from EMD Chemicals. Alternatively, levels of galectin-3 mRNA can be measured, again using methods known in the art and/or described herein, e.g., by quantitative PCR or Northern blotting analysis.

### EXAMPLES

The following examples are for purposes of illustration only and are not intended to limit the scope of the claims.

### EXAMPLE 1

Galectin-3 is a biomarker which has been implicated in a variety of biological processes important in heart failure including myofibroblast proliferation, fibrogenesis, tissue repair, cardiac remodeling, and inflammation. The addition of galectin-3 to the cardiac algorithm index score (either baseline or serial) was examined to see if it can improve the distinction between Type I and Type II MI patients.

The samples from patients were evaluated as Type I and Type 11 MI patients using a primary endpoint and endpoint adjudication described below. The Type I and Type II MI distinctions resulting from the cardiac algorithm index score, either baseline or serial, with the addition of Gal-3 were compared to these benchmarked classifications.

### Primary Outcome - Composite of death, nonfatal MI, and cardiac-related ED and hospital readmissions (all elements are adjudicated)

Participants were followed from 1 to 3 years after randomization to ascertain the occurrence of this endpoint.
a) Death includes all-cause mortality
b) Nonfatal MI was defined using the "Universal Definition" of MI: rise and/or fall of troponin with at least 1 value above the 99th percentile of the upper reference limit with at least one of the following: a) symptoms of ischemia, b) ECG changes indicative of new ischemia, c) Development of pathological Q waves on the ECG, and
d) Imaging evidence of new loss of viable myocardium or new regional wall motion abnormality. This endpoint does not include infarctions present at randomization as they could not relate to the study intervention.

### Endpoint adjudication

All components of the primary composite were adjudicated using a consensus of 3 cardiovascular and emergency care experts. Triggers for adjudication included a report of death, an uncertain vital status due to incomplete follow-up information, an elevated troponin value (excluding sequential rise and fall of values present at enrollment), hospital readmission, ED visit, recurrent cardiac testing after discharge, invasive angiography, and / or coronary revascularization. Endpoints adjudicated include the primary outcome, the secondary outcomes recurrent cardiac testing and cardiac-related ED visits, and the safety endpoint ACS after discharge.

To make the assessments, reviewers had access, either in summary form or actual data if needed, to the participant's index hospitalization admission and discharge records, results of relevant testing, follow-up call information, records obtained from follow-up, and study definitions.

### Performance of algorithm

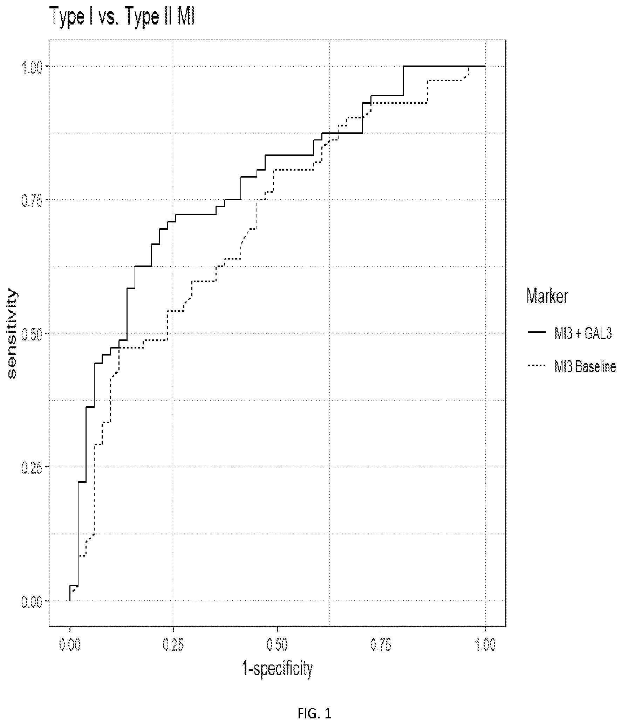

Concentrations of baseline Gal-3 were statistically significantly elevated in Type II MI patients vs. Type I MI patients. Adding baseline Gal-3 to the algorithm index score significantly improved AUC compared to the MI3 baseline score alone for Type I/Type II distinction (Table 1 and FIG. 1). This model resulted in an area under the curve (AUC) of 0.776 (95% CI 0.693, 0.858) for distinguishing Type I and Type II MI. This AUC shows a statistically significantly improvement compared to that of the baseline algorithm alone (p-value 0.0416 by the Delong method for comparing AUCs).

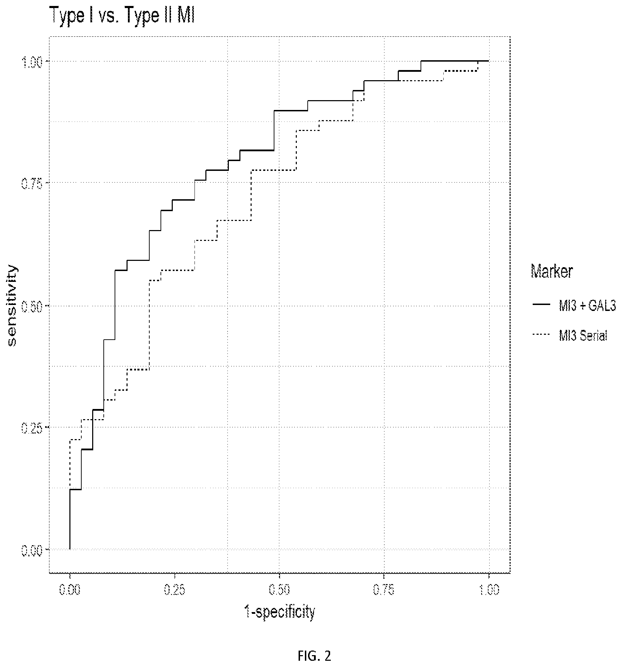

Adding baseline Gal-3 to the serial algorithm index score improved AUC compared to the MI3 serial score alone for Type I/Type II distinction (Table 2 and FIG. 2). This model resulted in an AUC of 0.791 (95% confidence interval (CI) 0.694, 0.888) for distinguishing Type I and Type II MI. Although not a statistically significant improvement in AUC, as with the baseline algorithm index described above, adding Gal-3 to the serial score resulted in a lower Akaike information criterion (AIC), 98.0, than the MI3 serial score model alone having an AIC of 109.2, indicating an improved fit (Table 2 and FIG. 2).

**Table 1**

| **Marker** | **AUC** | **95% CI Lower** | **95% CI Upper** |
|---|---|---|---|
| Baseline Algorithm Index Score | 0.704 | 0.611 | 0.797 |
| Baseline Algorithm Index Score plus Baseline Gal-3 | 0.776 | 0.693 | 0.858 |

**Table 2**

| **Marker** | **AUC** | **95% CI Lower** | **95% CI Upper** |
|---|---|---|---|
| Serial Algorithm Index Score | 0.721 | 0.614 | 0.829 |
| Serial Algorithm Index Score plus Baseline Gal-3 | 0.791 | 0.694 | 0.888 |

Tables 3 and 4 show the sensitivity, specificity, positive predictive value (PPV), and negative predictive value (NPV) at the optimal cutoff based on the minimum distance method for two models, one which uses the baseline Gal-3 plus the baseline index score and a second which uses the baseline Gal-3 plus a serial index score, respectively.

**Table 3**

| **Cutoff** | **Sensitivity** | **Specificity** | **PPV** | **NPV** |
|---|---|---|---|---|
| **0.58** | 0.722 | 0.745 | 0.800 | 0.655 |

**Table 4**

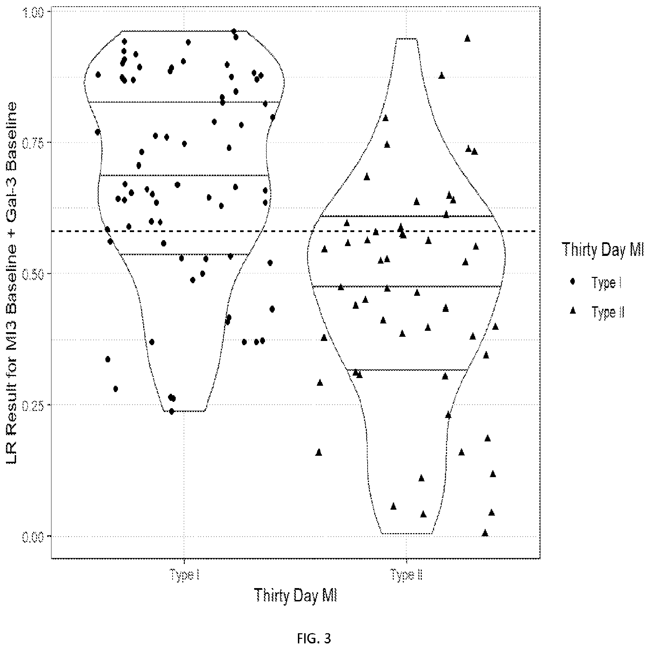

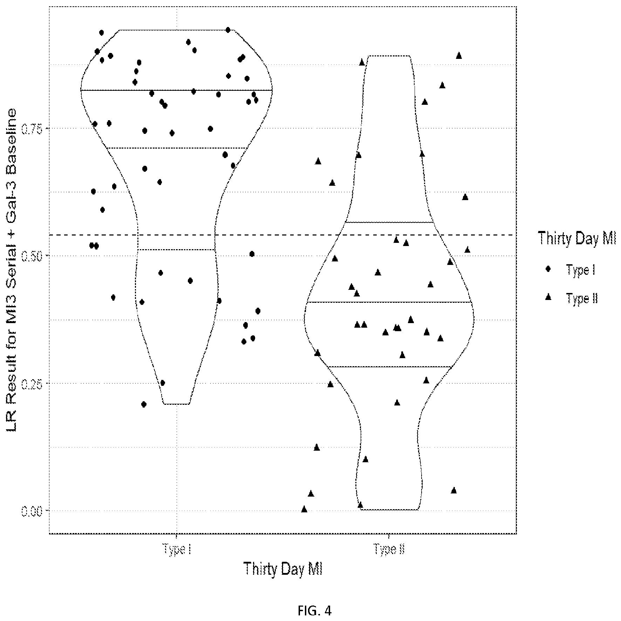

FIGS. 3 and 4 show predicted probability plots for both models with a horizontal line representing the optimal cutoff, which gives a visual representation of the model performance. As shown, the majority of Type I MI patients have values above the cutoff score (dotted line), whereas the majority of Type II MI patients have values below the cutoff score.

Although only a few exemplary embodiments have been described in detail, those skilled in the art will readily appreciate that many modifications are possible in the exemplary embodiments without materially departing from the novel teachings and advantages of this disclosure.

## Claims

1. A method of determining whether a subject suspected of having a myocardial infarction is experiencing a Type I myocardial infarction or a Type II myocardial infarction, the method comprising the steps of:
a) obtaining subject values for the subject, wherein said obtaining subject values comprises electronically receiving said subject values, wherein said subject values comprise:
i) a subject sex value;
ii) a subject age value;
iii) a subject initial cardiac troponin concentration from an initial sample from the subject; and
iv) a subject galectin-3 (Gal-3) concentration from an initial sample from the subject;
b) processing said subject's sex, age, and cardiac troponin value with a processing system such that an algorithm index score is determined for said subject, wherein said processing system comprises:
i) a computer processor, and
ii) non-transitory computer memory comprising one or more computer programs and a database, wherein said one or more computer programs comprise an additive tree algorithm,
wherein said database comprises at least M number of decision trees, wherein each individual decision tree comprises at least two pre-determined splitting variables and at least three pre-determined terminal node values,
wherein said at least two pre-determined splitting variables are a threshold initial cardiac troponin concentration value, a sex value, and/or an age value, wherein said one or more computer programs, in conjunction with said computer processor, is/are configured to:
i) apply said subject initial cardiac troponin concentration, said subject sex value, and/or said age value to said database to determine a terminal node value for each of said at least M number of decision trees, and
ii) apply said additive tree algorithm to: (a) determine a combined value from M number of said terminal node values, and (b) process said combined value to determine the algorithm index score that the subject is experiencing a myocardial infarction,
wherein M is an integer of at least 2,
c) reporting said algorithm index score for the subject determined by said processing system;
d) generating a probability score based on: i) the subject's Gal-3 concentration and ii) said algorithm index score; and
e) determining whether the subject has a Type I myocardial infarction or a Type II myocardial infarction.

2. A method of determining whether a subject suspected of having a myocardial infarction is experiencing a Type I myocardial infarction or a Type II myocardial infarction, the method comprising the steps of:
a) obtaining subject values for the subject, wherein said obtaining subject values comprises electronically receiving said subject values, wherein said subject values comprise:
i) a subject sex value;
ii) a subject age value;
iii) a subject initial cardiac troponin concentration from an initial sample from the subject;
iv) a subject galectin-3 (Gal-3) concentration from an initial sample from the subject; and
v) a first, second or a first and second subsequent cardiac troponin concentration from corresponding first and/or second subsequent samples from the subject;
b) processing said subject's sex, age, and cardiac troponin values with a processing system such that an algorithm index score is determined for said subject, wherein said processing system comprises:
i) a computer processor, and
ii) non-transitory computer memory comprising one or more computer programs and a database, wherein said one or more computer programs comprise: a rate of change algorithm and an additive tree algorithm,
wherein said database comprises at least M number of decision trees, wherein each individual decision tree comprises at least two pre-determined splitting variables and at least three pre-determined terminal node values,
wherein said at least two pre-determined splitting variables are: a threshold cardiac troponin rate of change value, a threshold initial cardiac troponin concentration value or a combination thereof; and a sex value and/or an age value, wherein said one or more computer programs, in conjunction with said computer processor, is/are configured to:
i) apply said rate of change algorithm to determine a subject cardiac troponin rate of change value from at least two of: said subject initial cardiac troponin concentration, said first subsequent cardiac troponin concentration, and said second subsequent cardiac troponin concentration,
ii) apply said subject cardiac troponin rate of change value, said subject initial cardiac troponin concentration, said subject sex value, and/or said age value to said database to determine a terminal node value for each of said at least M number of decision trees, and
iii) apply said additive tree algorithm to: (a) determine a combined value from M number of said terminal node values, and (b) process said combined value to determine the algorithm index score that the subject is experiencing a myocardial infarction,
wherein M is an integer of at least 2, and
c) reporting said algorithm index score for the subject determined by said processing system;
d) generating a probability score based on: i) the subject's Gal-3 concentration and ii) said algorithm index score; and
e) determining whether the subject has a Type I myocardial infarction or a Type II myocardial infarction based on the probability score.

3. The method of claim 1 or claim 2, wherein the subject is determined to have a Type I myocardial infarction based on the probability score.

4. The method of claim 1 or claim 2, wherein the subject is determined to have a Type II myocardial infarction based on the probability score.

5. The method of any of claims 1-4, wherein said obtaining subject values comprises receiving said subject values from a testing lab, from an analytical testing system, and/or from a hand-held or point of care testing device, optionally wherein said processing system further comprises said analytical testing system and/or said hand-held or point of care testing device.

6. The method of any of claims 1-5, wherein the initial cardiac troponin concentration, the first cardiac troponin concentration and/or the second cardiac troponin concentration is said obtained by performing a cardiac troponin detection assay, optionally wherein said cardiac troponin detection assay comprises an immunoassay, and/or wherein the cardiac troponin detection assay is a single molecule detection assay.

7. The method of any of claims 1-6, wherein the cardiac troponin is cardiac troponin I (cTnl).

8. The method of any of claims 1-7, wherein the cardiac troponin is cardiac troponin T (cTnT).

9. The method of any of claims 1-8, wherein the Gal-3 concentration is obtained by performing a Gal-3 detection assay, optionally wherein said Gal-3 detection assay comprises an immunoassay, and/or wherein the Gal-3 detection assay is a single molecule detection assay.

10. The method of any of claims 1-9, further comprising manually or automatically inputting said subject values into said processing system.

11. The method of any of claims 1-10, wherein said subject is a human.

12. The method of any of claims 1-11, wherein said initial samples from said subject comprises a blood, serum, or plasma sample.

13. The method any of claims 1-11, wherein said first and/or second subsequent samples comprise blood, serum, or plasma samples.

14. The method of any of claims 1-13, wherein said M decision trees is at least 100 different decision trees

15. The method of any of claims 1-14, wherein said M decision trees is at least 800 different decision trees.

## Patentansprüche

1. Verfahren zum Bestimmen, ob ein Patient, bei dem vermutet wird, dass er einen Myokardinfarkt hat, einen Myokardinfarkt Typ I oder einen Myokardinfarkt Typ II aufweist, wobei das Verfahren die folgenden Schritte umfasst:
a) Erhalten von Patientenwerten für den Patienten, wobei das Erhalten von Patientenwerten das elektronische Empfangen der Patientenwerte umfasst, wobei die Patientenwerte Folgendes umfassen:
i) einen Wert des Patienten-Geschlechts;
ii) einen Wert des Patienten-Alters;
iii) eine anfängliche Herztroponinkonzentration des Patienten aus einer anfänglichen Probe des Patienten; und
iv) eine Galectin-3 (Gal-3)-Konzentration des Patienten aus einer anfänglichen Probe des Patienten;
b) Verarbeiten des Geschlechts-, Alters- und Herztroponinwerts des Patienten mit einem Verarbeitungssystem, so dass ein Algorithmus-Index-Score für den Patienten bestimmt wird, wobei das Verarbeitungssystem umfasst:
i) einen Computerprozessor, und
ii) nichtflüchtigen Computerspeicher, der ein oder mehrere Computerprogramme und eine Datenbank umfasst, wobei das eine oder die mehreren Computerprogramme einen additiven Baumalgorithmus umfassen,
wobei die Datenbank mindestens M Anzahl von Entscheidungsbäumen umfasst, wobei jeder einzelne Entscheidungsbaum mindestens zwei vorbestimmte Aufteilungsvariablen und mindestens drei vorbestimmte Endknotenwerte umfasst,
wobei die mindestens zwei vorbestimmten Aufteilungsvariablen ein Schwellenwert für die anfängliche Herztroponinkonzentration, ein Geschlechtswert und/oder ein Alterswert sind, wobei das eine oder die mehreren Computerprogramm(e) in Verbindung mit dem Computerprozessor konfiguriert ist/sind zum:
i) Anwenden der anfänglichen Herztroponinkonzentration des Patienten, des Geschlechtswerts des Patienten und/oder des Alterswerts auf die Datenbank, um einen Endknotenwert für jeden der mindestens M Anzahl von Entscheidungsbäumen zu bestimmen, und
ii) Anwenden des additiven Baumalgorithmus zum: (a) Bestimmen eines kombinierten Werts aus der M-Anzahl der Endknotenwerte und (b) Verarbeiten des kombinierten Werts zum Bestimmen des Algorithmus-Index-Score, dass der Patient einen Myokardinfarkt erfährt,
wobei M eine ganze Zahl von mindestens 2 ist,
c) Berichten des Algorithmus-Index-Score für den Patienten, der durch das Verarbeitungssystem bestimmt wurde;
d) Erzeugen eines Wahrscheinlichkeits-Score basierend auf: i) der Gal-3-Konzentration des Patienten und ii) dem Algorithmus-Index-Score; und
e) Bestimmen, ob der Patient einen Typ-I-Myokardinfarkt oder einen Typ-II-Myokardinfarkt aufweist.

2. Verfahren zum Bestimmen, ob ein Patient, bei dem vermutet wird, dass er einen Myokardinfarkt hat, einen Myokardinfarkt Typ I oder einen Myokardinfarkt Typ II aufweist, wobei das Verfahren die folgenden Schritte umfasst:
a) Erhalten von Patientenwerten für den Patienten, wobei das Erhalten von Patientenwerten das elektronische Empfangen der Patientenwerte umfasst, wobei die Patientenwerte Folgendes umfassen:
i) einen Wert des Patienten-Geschlechts;
ii) einen Wert des Patienten-Alters;
iii) eine anfängliche Herztroponinkonzentration des Patienten aus einer anfänglichen Probe des Patienten;
iv) eine Galectin-3(Gal-3)-Konzentration des Patienten aus einer anfänglichen Probe des Patienten; und
v) eine erste, eine zweite oder eine erste und eine zweite nachfolgende Herztroponinkonzentration aus entsprechenden ersten und/oder zweiten nachfolgenden Proben aus dem Patienten;
b) Verarbeiten der Geschlechts-, Alters- und Herztroponinwerte des Patienten mit einem Verarbeitungssystem, so dass ein Algorithmus-Index-Score für den Patienten bestimmt wird, wobei das Verarbeitungssystem umfasst:
i) einen Computerprozessor, und
ii) nichtflüchtigen Computerspeicher, der ein oder mehrere Computerprogramme und eine Datenbank umfasst, wobei das eine oder die mehreren Computerprogramme umfassen: einen Änderungsratenalgorithmus und einen additiven Baumalgorithmus,
wobei die Datenbank mindestens M Anzahl von Entscheidungsbäumen umfasst, wobei jeder einzelne Entscheidungsbaum mindestens zwei vorbestimmte Aufteilungsvariablen und mindestens drei vorbestimmte Endknotenwerte umfasst,
wobei die mindestens zwei vorbestimmten Aufteilungsvariablen Folgende sind: ein Schwellenwert für den Herztroponin-Änderungsratenwert, ein Schwellenwert für den anfängliche-Herztroponinkonzentration-Wert oder eine Kombination davon; und ein Geschlechtswert und/oder ein Alterswert, wobei das eine oder die mehreren Computerprogramm(e) in Verbindung mit dem Computerprozessor konfiguriert ist/sind zum:
i) Anwenden des Änderungsratenalgorithmus, um einen Herztroponin-Änderungsratenwert des Patienten aus mindestens zwei von Folgendem zu bestimmen: der anfänglichen Herztroponinkonzentration des Patienten, der ersten nachfolgenden Herztroponinkonzentration und der zweiten nachfolgenden Herztroponinkonzentration,
ii) Anwenden des Herztroponin-Änderungsratenwerts des Patienten, der anfänglichen Herztroponinkonzentration des Patienten, des Geschlechtswerts des Patienten und/oder des Alterswerts auf die Datenbank, um einen Endknotenwert für jeden der mindestens M Anzahl von Entscheidungsbäumen zu bestimmen, und
iii) Anwenden des additiven Baumalgorithmus zum: (a) Bestimmen eines kombinierten Werts aus der M-Anzahl der Endknotenwerte und (b) Verarbeiten des kombinierten Werts zum Bestimmen des Algorithmus-Index-Score, dass der Patient einen Myokardinfarkt erfährt,
wobei M eine ganze Zahl von mindestens 2 ist, und
c) Berichten des Algorithmus-Index-Score für den Patienten, der durch das Verarbeitungssystem bestimmt wurde;
d) Erzeugen eines Wahrscheinlichkeits-Score basierend auf: i) der Gal-3-Konzentration des Patienten und ii) dem Algorithmus-Index-Score; und
e) Bestimmen, ob der Patient einen Typ-I-Myokardinfarkt oder einen Typ-II-Myokardinfarkt hat, basierend auf dem Wahrscheinlichkeits-Score.

3. Verfahren nach Anspruch 1 oder Anspruch 2, wobei basierend auf dem Wahrscheinlichkeits-Score bestimmt wird, dass der Patient einen Typ-I-Myokardinfarkt aufweist.

4. Verfahren nach Anspruch 1 oder Anspruch 2, wobei basierend auf dem Wahrscheinlichkeits-Score bestimmt wird, dass der Patient einen Typ-II-Myokardinfarkt aufweist.

5. Verfahren nach einem der Ansprüche 1-4, wobei das Erhalten von Patientenwerten das Empfangen der Patientenwerte von einem Testlabor, von einem analytischen Testsystem und/oder von einer Hand- oder Pflegestellen-Testvorrichtung umfasst, wobei optional das Verarbeitungssystem ferner das analytische Testsystem und/oder die Hand- oder Pflegestellen-Testvorrichtung umfasst.

6. Verfahren nach einem der Ansprüche 1-5, wobei die anfängliche Herztroponinkonzentration, die erste Herztroponinkonzentration und/oder die zweite Herztroponinkonzentration durch Durchführen eines Herztroponin-Nachweisassays erhalten werden, wobei optional der Herztroponin-Nachweisassay einen Immunassay umfasst und/oder wobei der Herztroponin-Nachweisassay ein Einzelmolekül-Nachweisassay ist.

7. Verfahren nach einem der Ansprüche 1-6, wobei es sich bei dem Herztroponin um kardiales Troponin I (cTnI) handelt.

8. Verfahren nach einem der Ansprüche 1-7, wobei es sich bei dem Herztroponin um kardiales Troponin T (cTnT) handelt.

9. Verfahren nach einem der Ansprüche 1-8, wobei die Gal-3-Konzentration durch Durchführen eines Gal-3-Nachweisassays erhalten wird, wobei der Gal-3-Nachweisassay optional einen Immunassay umfasst und/oder wobei der Gal-3-Nachweisassay ein Einzelmolekül-Nachweisassay ist.

10. Verfahren nach einem der Ansprüche 1-9, ferner umfassend manuelles oder automatisches Eingeben der Patientenwerte in das Verarbeitungssystem.

11. Verfahren nach einem der Ansprüche 1-10, wobei der Patient ein Mensch ist.

12. Verfahren nach einem der Ansprüche 1-11, wobei die anfänglichen Proben aus dem Patienten
eine Blut-, Serum- oder Plasmaprobe umfassen.

13. Verfahren nach einem der Ansprüche 1-11, wobei die erste und/oder die zweite nachfolgende Probe Blut-, Serum- oder Plasmaproben umfassen.

14. Verfahren nach einem der Ansprüche 1-13, wobei die M Entscheidungsbäume mindestens 100 verschiedene Entscheidungsbäume sind.

15. Verfahren nach einem der Ansprüche 1-14, wobei die M Entscheidungsbäume mindestens 800 verschiedene Entscheidungsbäume sind.

## Revendications

1. Procédé pour déterminer si un sujet suspecté d'avoir un infarctus du myocarde subit un infarctus du myocarde de type I ou un infarctus du myocarde de type II, le procédé comprenant les étapes de :
a) obtention de valeurs du sujet pour le sujet, dans lequel ladite obtention de valeurs du sujet comprend la réception électronique desdites valeurs du sujet, lesdites valeurs du sujet comprenant :
i) une valeur de sexe du sujet ;
ii) une valeur d'âge du sujet ;
iii) une concentration initiale de troponine cardiaque du sujet à partir d'un échantillon initial du sujet ; et
iv) une concentration de galectine 3 (Gal-3) du sujet à partir d'un échantillon initial du sujet ;
b) le traitement desdites valeurs du sexe, de l'âge et de troponine cardiaque du sujet par un système de traitement de sorte qu'un score d'indice d'algorithme soit déterminé pour ledit sujet, ledit système de traitement comprenant :
i) un processeur informatique, et
ii) une mémoire informatique non transitoire comprenant un ou plusieurs programmes informatiques et une base de données, dans lequel lesdits un ou plusieurs programmes informatiques comprennent un algorithme d'arbre additif,
dans lequel ladite base de données comprend au moins un nombre M d'arbres de décision, chaque arbre de décision individuel comprenant au moins deux variables de division prédéterminées et au moins trois valeurs de nœuds terminaux prédéterminées,
dans lequel lesdites au moins deux variables de division prédéterminées sont une valeur seuil de concentration initiale de troponine cardiaque, une valeur de sexe et/ou une valeur d'âge, dans lequel lesdits un ou plusieurs programmes informatiques, en conjonction avec ledit processeur informatique, est/sont configuré(s) pour :
i) appliquer ladite concentration initiale de troponine cardiaque du sujet, ladite valeur de sexe du sujet et/ou ladite valeur d'âge à ladite base de données pour déterminer une valeur de nœud terminal pour chacun dudit au moins nombre M d'arbres de décision, et
ii) appliquer ledit algorithme d'arbre additif pour : (a) déterminer une valeur combinée à partir du nombre M desdites valeurs de nœuds terminaux, et (b) traiter ladite valeur combinée pour déterminer le score d'indice d'algorithme que le sujet subit un infarctus du myocarde, dans lequel M est un entier d'au moins 2,
c) rapporter ledit score d'indice d'algorithme pour le sujet déterminé par ledit système de traitement ;
d) générer un score de probabilité basé sur : i) la concentration de Gal-3 du sujet et ii) ledit score d'indice d'algorithme ; et
e) déterminer si le sujet a un infarctus du myocarde de type I ou un infarctus du myocarde de type II.

2. Procédé pour déterminer si un sujet suspecté d'avoir un infarctus du myocarde subit un infarctus du myocarde de type I ou un infarctus du myocarde de type II, le procédé comprenant les étapes de :
a) obtention de valeurs du sujet pour le sujet, dans lequel ladite obtention de valeurs du sujet comprend la réception électronique desdites valeurs du sujet, lesdites valeurs du sujet comprenant :
i) une valeur de sexe du sujet ;
ii) une valeur d'âge du sujet ;
iii) une concentration initiale de troponine cardiaque chez un sujet à partir d'un échantillon initial du sujet;
iv) une concentration de galectine-3 (Gal-3) du sujet à partir d'un échantillon initial du sujet ; et
v) une première, une deuxième ou une première et une deuxième concentration de troponine cardiaque suivantes à partir de premier et/ou deuxième échantillons suivants correspondants du sujet ;
b) traitement desdites valeurs de sexe, d'âge et de troponine cardiaque du sujet par un système de traitement de sorte qu'un score d'indice d'algorithme soit déterminé pour ledit sujet, ledit système de traitement comprenant:
i) un processeur informatique, et
ii) une mémoire informatique non transitoire comprenant un ou plusieurs programmes informatiques et une base de données, dans lequel lesdits un ou plusieurs programmes informatiques comprennent : un algorithme de taux de changement et un algorithme d'arbre additif,
dans lequel ladite base de données comprend au moins un nombre M d'arbres de décision, chaque arbre de décision individuel comprenant au moins deux variables de division prédéterminées et au moins trois valeurs de nœuds terminaux prédéterminées,
dans lequel lesdites au moins deux variables de division prédéterminées sont : une valeur seuil de taux de changement de troponine cardiaque, une valeur seuil de concentration initiale de troponine cardiaque ou une combinaison de celles-ci ; et une valeur de sexe et/ou une valeur d'âge, dans lequel lesdits un ou plusieurs programmes informatiques, en conjonction avec ledit processeur informatique, est/sont configuré(s) pour :
i) appliquer ledit algorithme de taux de changement pour déterminer une valeur de taux de changement de troponine cardiaque du sujet à partir d'au moins deux des valeurs suivantes : ladite concentration initiale de troponine cardiaque du sujet, ladite première concentration subséquente de troponine cardiaque et ladite deuxième concentration subséquente de troponine cardiaque,
ii) appliquer ladite valeur de changement de taux de troponine cardiaque du sujet, ladite concentration initiale de troponine cardiaque du sujet, ladite valeur de sexe du sujet et/ou ladite valeur d'âge à ladite base de données pour déterminer une valeur de nœud terminal pour chacun dudit au moins nombre M d'arbres de décision, et
iii) appliquer ledit algorithme d'arbre additif pour : (a) déterminer une valeur combinée à partir du nombre M desdites valeurs de nœuds terminaux, et (b) traiter ladite valeur combinée pour déterminer le score d'indice d'algorithme que le sujet subit un infarctus du myocarde, dans lequel M est un entier d'au moins 2, et
c) rapporter ledit score d'indice d'algorithme pour le sujet déterminé par ledit système de traitement ;
d) générer un score de probabilité basé sur : i) la concentration de Gal-3 du sujet et ii) ledit score d'indice d'algorithme ; et
e) déterminer si le sujet a un infarctus du myocarde de type I ou un infarctus du myocarde de type II sur la base du score de probabilité.

3. Procédé selon la revendication 1 ou la revendication 2, dans lequel le sujet est déterminé comme ayant un infarctus du myocarde de type I sur la base du score de probabilité.

4. Procédé selon la revendication 1 ou la revendication 2, dans lequel le sujet est déterminé comme ayant un infarctus du myocarde de type II sur la base du score de probabilité.

5. Procédé selon l'une quelconque des revendications 1 à 4, dans lequel ladite obtention de valeurs du sujet comprend la réception desdites valeurs du sujet d'un laboratoire d'essai, d'un système d'essai analytique et/ou d'un dispositif d'essai portatif ou sur le lieu de soins, éventuellement ledit système de traitement comprenant en outre ledit système d'essai analytique et/ou ledit dispositif d'essai portatif ou sur le lieu de soins.

6. Procédé selon l'une quelconque des revendications 1 à 5, dans lequel la concentration initiale de troponine cardiaque, la première concentration cardiaque de troponine et/ou la seconde concentration cardiaque de troponine sont lesdites obtenues en effectuant un dosage de détection de troponine cardiaque, éventuellement dans lequel ledit dosage de détection de troponine cardiaque comprend un dosage immunologique, et/ou dans lequel le dosage de détection de troponine cardiaque est un dosage de détection de molécule unique.

7. Procédé selon l'une quelconque des revendications 1 à 6, dans lequel la troponine cardiaque est la troponine cardiaque I (cTnI).

8. Procédé selon l'une quelconque des revendications 1 à 7, dans lequel la troponine cardiaque est la troponine cardiaque T (cTnT).

9. Procédé selon l'une quelconque des revendications 1 à 8, dans lequel la concentration de Gal-3 est obtenue en effectuant un essai de détection de Gal-3, facultativement dans lequel ledit essai de détection de Gal-3 comprend un essai immunologique, et/ou dans lequel l'essai de détection de Gal-3 est un essai de détection de molécule unique.

10. Procédé selon l'une quelconque des revendications 1 à 9, comprenant en outre l'entrée manuelle ou automatique desdites valeurs du sujet dans ledit système de traitement.

11. Procédé selon l'une quelconque des revendications 1 à 10, dans lequel ledit sujet est un humain.

12. Procédé selon l'une quelconque des revendications 1 à 11, dans lequel lesdits échantillons initiaux provenant dudit sujet
comprennent un échantillon de sang, de sérum ou de plasma.

13. Procédé selon l'une quelconque des revendications 1 à 11, dans lequel lesdits premier et/ou second échantillons suivants comprennent des échantillons de sang, de sérum ou de plasma.

14. Procédé selon l'une quelconque des revendications 1 à 13, dans lequel lesdits M arbres de décision sont au moins 100 arbres de décision différents

15. Procédé selon l'une quelconque des revendications 1 à 14, dans lequel lesdits M arbres de décision sont au moins 800 arbres de décision différents.
